# EUROPEAN PATENT APPLICATION

(11) **EP 2 258 259 A1**
(43) Date of publication of application: **08.12.2010**
(21) Application number: 10005507.8
(22) Date of filing: 27.05.2010
(51) Int. Cl.: A61B 5/022

(54) **Blood pressure cuff**

(30) Priority: 03.06.2009 JP 2009133880
(71) Applicant: Panasonic Electric Works Co., Ltd., Kadoma-shi Osaka (JP)
(72) Inventor: Kanetsuna, Yoshitoshi, Kadoma-shi Osaka (JP); Kojima, Takeshi, Kadoma-shi Osaka (JP); Fumuro, Shinichi, Kadoma-shi Osaka (JP)
(74) Representative: Samson & Partner

(57) **Abstract**

A blood pressure cuff includes an inflatable air bag for restricting blood flow; an elastic circular arc-shaped support plate arranged at an outer peripheral side of the air bag; a cover strip for surrounding the air bag and the support plate; a ring fixed to the support plate, the ring being used to fold a leading portion; and a fastener for releasably engaging the folded-over leading portion. The fastener includes hook and loop portions that are mixedly arranged on a same surface at a substantially equal occupancy ratio, and the fastener is set to have a length which allows the engagement of the fastener to be carried out at a location close to the ring, in either case that the cover strip has an estimated maximum inner diameter of the cover strip or an estimated minimum inner diameter thereof.

## Description

### Field of the Invention

The present invention relates to a blood pressure cuff which is placed around an arm or wrist of a human body to restrict a blood flow in a blood pressure measurement.

### Background of the Invention

In a blood pressure measurement, a blood pressure cuff is employed to restrict blood flow by wrapping the cuff around an upper arm or wrist of a user and inflating an air bag inside the cuff to occlude a corresponding artery.

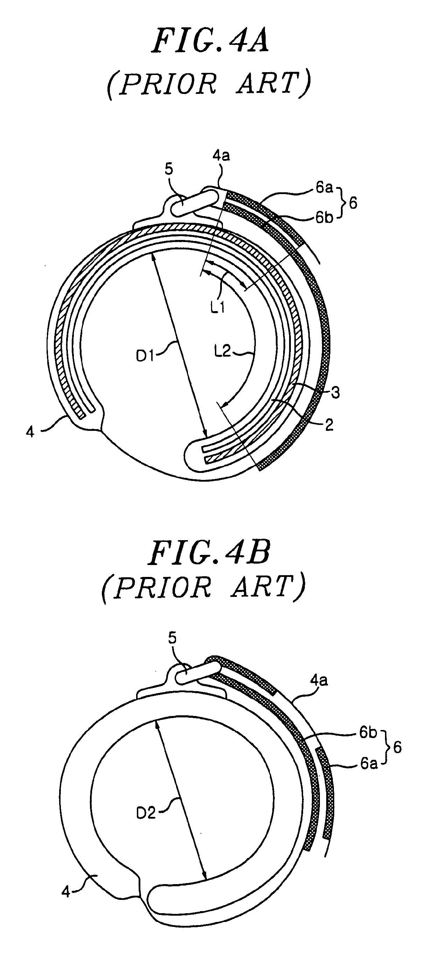

Such a blood pressure cuff, as shown in Fig. 4A, includes an inflatable air bag 2 for restricting blood flow; an elastic support plate 3 arranged at an outer peripheral side of the air bag 2, the support plate 3 being bendable into a circular arc shape; a cover strip 4 for surrounding the air bag 2 and the support plate 3; a ring 5 fixed to the support plate 3, the ring 5 being used to fold over a leading part 4a of the cover strip 4; and a fastener 6 for releasably engaging the folded-over leading part 4a (see, e.g., Japanese Patent Application Publication No. 2009-077824).

The fastener 6 includes a hook portion 6a and a loop portion 6b. When the hook and the loop portion 6a and 6b are strongly pressed to each other, they are engaged with each other such that it becomes difficult to disengage the hook portion 6a and the loop portion 6b from each other. However, if a user strongly detaches the hook portion 6a from the loop portion 6b with the hand, the hook portion 6a can be disengaged from the loop portion 6b. The fastener 6 is also referred to as "mechanical fastener."

The hook portion 6a of the fastener 6 is provided at a leading end portion of the leading part 4a of the cover strip 4 and has a length L1. The loop portion 6b of the fastener 6 is provided at a trailing end portion of the leading part 4a of the cover strip 4 and has a length L2.

Meanwhile, an upper arm of a user may be thick or thin. In case the cuff is wrapped around a thick upper arm of a user, an estimated maximum inner diameter D1 of the cover strip 4 is increased (about 34 cm in circumference length) as shown in Fig. 4A. In case the cuff is wrapped around a thin upper arm of a user, an estimated minimum inner diameter D2 of the cover strip 4 is decreased (about 20 cm in circumference length) as shown in Fig. 4B.

Moreover, in case the cover strip 4 is wrapped around the thick upper arm as shown in Fig. 4A, the hook portion 6a of the fastener 6 of the leading part 4a can be engaged with the loop portion 6b at a location close to the ring 5.

When, however, the cover strip 4 is wrapped around the thin upper arm as shown in Fig. 4B, the hook portion 6a of the fastener 6 of the leading part 4a is engaged with the loop portion 6b at a location remote from the ring 5.

For that reason, it is required to increase the length L2 of the loop portion 6b while the length L1 of the loop portion 6a is short, which makes the overall length of the cover strip 4 increased. Accordingly, it becomes difficult to wrap the blood pressure cuff around the thin upper arm.

### Summary of the Invention

In view of the above, the present invention provides a blood pressure cuff capable of being easily placed around an upper arm of a user to stably restrict blood flow regardless of whether the upper arm is thick or thin.

In accordance with an embodiment of the present invention, there is provided a blood pressure cuff including an inflatable air bag for restricting blood flow; an elastic circular arc-shaped support plate arranged at an outer peripheral side of the air bag; a cover strip for surrounding the air bag and the support plate; a ring fixed to the support plate, the ring being used to fold a leading portion of the cover strip; and a fastener for releasably engaging the folded-over leading side. The fastener includes hook and loop portions that are mixedly arranged on a same surface at a substantially equal occupancy ratio, and the fastener is set to have a length which allows the engagement of the fastener to be carried out at a location close to the ring, in either case that the cover strip has an estimated maximum inner diameter or an estimated minimum inner diameter.

With such configuration, it is possible to make the fastener engaged at the location close to the ring regardless of whether the upper arm is thick or thin and there is no case that the engagement of the fastener is carried out at a location remote from the ring. For that reason, even when the blood pressure cuff is placed around a thin upper arm, it is easy to place the blood pressure cuff around the upper arm, which allows stable restriction of the blood flow. Moreover, since the length of the expensive fastener becomes shorter, the price of the blood pressure cuff is decreased.

In accordance with a modification of the above embodiment, the hook and the loop portions of the fastener may alternately be arranged in the lengthwise direction of the cover strip, obliquely with regard to the width direction thereof.

The modification of the above embodiment may be easily embodied by crosscutting and alternately attaching sheets of the conventional hook and loop portions. This results in a low manufacturing cost.

### Brief Description of the Drawings

The objects and features of the present invention will become apparent from the following description of embodiments, given in conjunction with the accompanying drawings, in which:
Fig. 1 is a perspective view showing a blood pressure cuff in accordance with an embodiment of the present invention;
Figs. 2A and 2B are cross sectional views showing the blood pressure cuff shown in Fig. 1 when it is placed around a thick and a thin upper arm, respectively;
Fig. 3A is an enlarged perspective view showing a fastener of the blood pressure cuff shown in Fig. 1;
Fig. 3B is a plan view showing a fastener in accordance with a modification of the present embodiment; and
Figs. 4A and 4B are cross sectional views showing a conventional blood pressure cuff when it is wrapped around a thick and a thin upper arm, respectively.

### Detailed Description of the Embodiments

An embodiment of the present invention will now be described with reference to the accompanying drawings which form a part hereof.

In the following description and drawings, components having the substantially same configuration and function as those described in "Background of the Invention" section are denoted by like reference characters and, thus, redundant description thereof will be omitted herein.

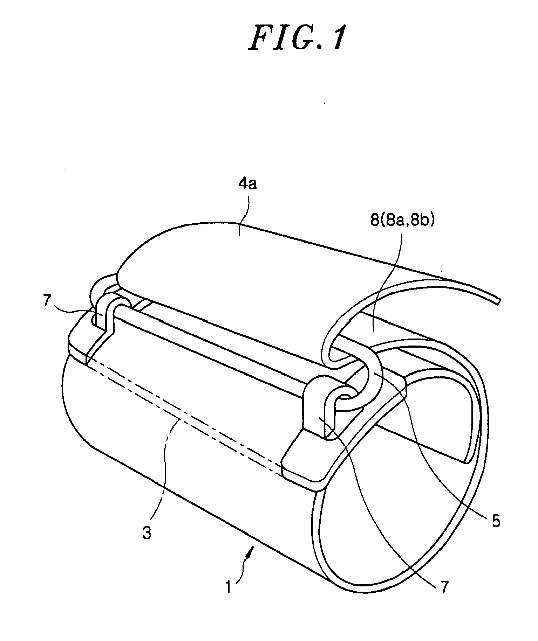

Fig. 1 shows a blood pressure cuff 1. The blood pressure cuff 1 is placed around an upper arm (or wrist) of a user by inserting it into the cuff 1.

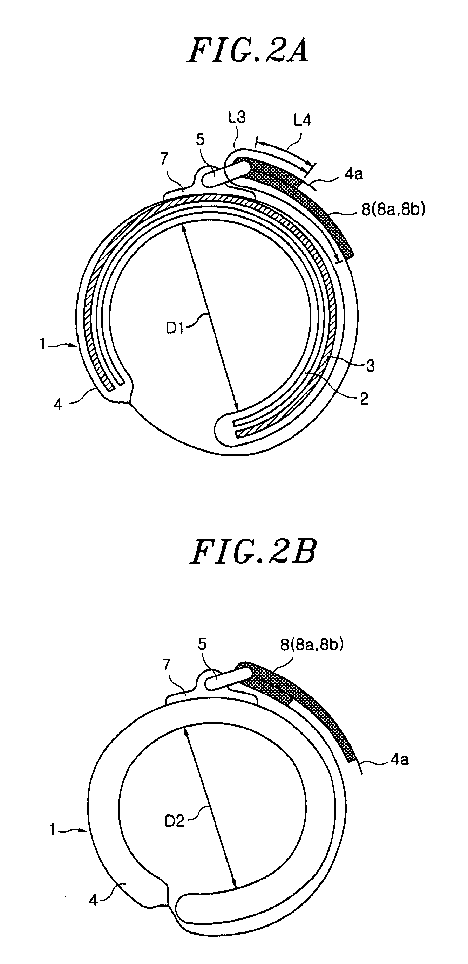

As shown in Fig. 2A, the blood pressure cuff 1 includes an air bag 2 for restricting blood flow, the air bag 2 being inflated by supplying air thereto by an air pump or like and deflated by exhausting the air therefrom through an exhaust valve or the like; and an elastic circular arc-shaped support plate (C-shaped clip) 3 arranged at an outer peripheral side of the air bag 2. The air bag 2 and the support plate 3 are surrounded by and accommodated in a cover strip 4.

A pair of mounting bases 7 is fixed at opposite portions on an outer peripheral surface of the support plate 3 in a width direction by using screws or the like extending through the cover strip 4. An elliptical ring 5 formed of a metal bar member is rotatably held through the opposite mounting bases 7. The ring 5 is used to fold over a leading part 4a of the cover strip 4.

A fastener 8 is attached on the outer surface of the leading part 4a and serves to releasably engage the folded-over leading part 4a. The fastener 8 will be described later in detail.

When the blood pressure cuff 1 is placed around an upper arm of a user, the user inserts the hand into the blood pressure cuff 1 such that the blood pressure cuff 1 is placed around the upper arm while the support plate 3 maintaining the blood pressure cuff 1 in the substantially cylindrical shape is widened against its elastic force. Thereafter, the blood pressure cuff 1 is adjusted such that the ring 5 is positioned at the side of the upper arm that faces the wrist when the wrist is raised toward the upper arm. Then, the blood pressure cuff 1 is tightened by pulling the leading part 4a of the cover strip 4 folded through the ring 5 and fixed by using the fastener 8 provided on the leading part 4a.

As known in the art, by supplying air to inflate the air bag 2, the brachial artery is occluded and the blood flow is restricted. Then, the blood pressure is measured as the air bag 2 is deflated by slowly exhausting the air therefrom.

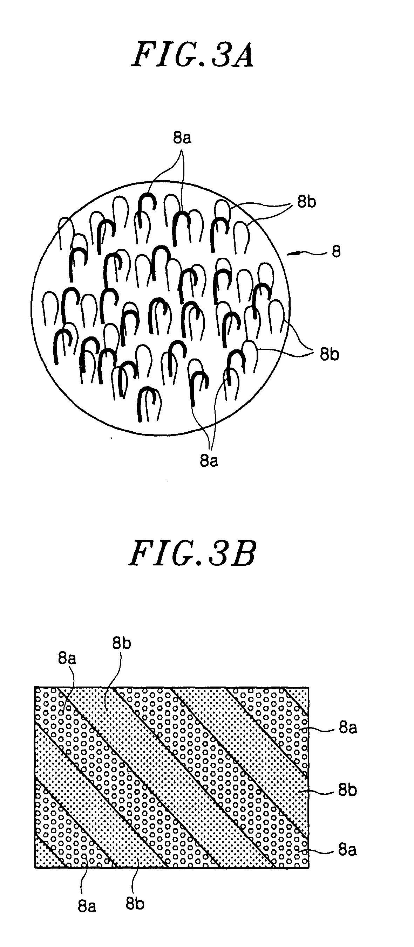

As shown in Fig. 3A, the fastener 8 includes hook and loop portions 8a and 8b that are densely mixed on the same surface at a substantially equal occupation ratio.

The fastener 8 is set to have a length L3 which allows the engagement of the fastener 8 to be carried out at a location close to the ring 5, in either case that the cover strip 4 has an estimated maximum inner diameter D1 (about 34 cm in circumference length) for a thick upper arm as shown in Fig. 2A or an estimated minimum inner diameter D2 (about 20 cm in circumference length) for a thin upper arm as shown in Fig. 2B. A length L4 of the fastener 8 engaged at a location close to the ring 5 is preferably set to be, e.g., about 3 to 5 cm.

The above-mentioned blood pressure cuff 1 has the fastener 8 including the hook and loop portions 8a and 8b that are densely mixed on the same surface, and the fastener 8 is set to have the length L3 which allows the engagement of the fastener 8 to be carried out at the location close to the ring 5, in either case that the cover strip 4 has the estimated maximum inner diameter D1 or the estimated minimum inner diameter D2.

Accordingly, it is possible to make the fastener 8 engaged at the location close to the ring 5 regardless of whether the upper arm is thick or thin and there is no case that the engagement of the fastener 8 is carried out at a location remote from the ring 5.

For that reason, even when the blood pressure cuff 1 is placed around a thin upper arm, it is easy to place the blood pressure cuff 1 around the upper arm, which allows stable restriction of the blood flow. Moreover, since the length L3 of the expensive fastener 8 becomes shorter (about (L1 + L2)/2, L1 and L2 respectively denoting the lengths of the hook and the loop portion 6a and 6b of the fastener 6 in the conventional case shown in Figs. 4A and 4B), the price of the blood pressure cuff 1 is decreased.

Fig. 3B shows the fastener 8 in accordance with a modification of the above embodiment. The hook and the loop portions 8a and 8b of the fastener 8 are alternately arranged in the lengthwise direction of the cover strip 4, obliquely with regard to the width direction thereof.

In accordance with the modification of the above embodiment, sheets of the conventional hook and loop portions 6a and 6b may be crosscut and alternately attached, which results in a low manufacturing cost.

While the invention has been shown and described with respect to the embodiments, it will be understood by those skilled in the art that various changes and modifications may be made without departing from the scope of the invention as defined in the following claims.

## Claims

1. A blood pressure cuff comprises:
an inflatable air bag for restricting blood flow;
an elastic circular arc-shaped support plate arranged at an outer peripheral side of the air bag;
a cover strip for surrounding the air bag and the support plate;
a ring fixed to the support plate, the ring being used to fold a leading part of the cover strip; and
a fastener for releasably engaging the folded-over leading part,
wherein the fastener includes hook and loop portions that are mixedly arranged on a same surface at a substantially equal occupancy ratio, and the fastener is set to have a length which allows the engagement of the fastener to be carried out at a location close to the ring, in either case that the cover strip has an estimated maximum inner diameter or an estimated minimum inner diameter.

2. The blood pressure cuff of claim 1, wherein the hook and loop portions of the fastener are alternately arranged in a lengthwise direction of the cover strip, obliquely with regard to a width direction thereof.
